# EUROPÄISCHE PATENTANMELDUNG

(11) **EP 1 688 136 A1**
(43) Veröffentlichungstag der Anmeldung: **09.08.2006**
(21) Anmeldenummer: 06002012.0
(22) Anmeldetag: 01.02.2006
(51) Int. Cl.: A61K 31/122

(54) **Hydrophile Biochinon-Verbindung, Verfahren zu ihrer Herstellung und ihre Verwendung**

(30) Priorität: 03.02.2005 DE 102005005054
(71) Anmelder: Schubert, Karl Heinz, Ing., 5061 Elsbethen (AT); Schall, Ronald, 5020 Salzburg (AT); Kastler, Severin, 86150 Augsburg (DE)
(72) Erfinder: Schubert, Karl Heinz, Ing., 5061 Elsbethen (AT); Schall, Ronald, 5020 Salzburg (AT); Kastler, Severin, 86150 Augsburg (DE)
(74) Vertreter: Gallo, Wolfgang

(57) **Zusammenfassung**

Es wird eine Verbindung mit einem an einen Zucker gebundenen Biochinon, ein Verfahren zu ihrer Herstellung, eine Zusammensetzung mit dieser Verbindung und die Verwendung dieser Verbindung bzw. Zusammensetzung für die Herstellung von pharmazeutischen und kosmetischen Formulierungen bzw. Nahrungsergänzungsmitteln zur Verfügung gestellt.

## Beschreibung

Die Erfindung beschäftigt sich mit Verbindungen aus Biochinon mit Einfach-, Mehrfachzuckern mit hydrophilen Eigenschaften, die mittels eines mechanischen Verfahrens ohne notwendige Zugabe weiterer chemischer Begleitstoffe hergestellt werden kann.

Biochinone sind Chinonderivate mit einer langen Isoprenoidkette, wobei zu dieser Gruppe u.a. Ubichinone, Bovichinone, Plastochinone und K-Vitamine gehören.

Ubichinone (auch Coenzym Q genannt) sind ubiquitär in Organismen auftretende Substanzen. In fast allen Organismen können Ubichinone nachgewiesen werden, wobei gram-positive Bakterien und Cyanobakterien eine Ausnahme bilden. Ubichinone werden in Abhängigkeit von der Zahl der in der Seitenkette verknüpften Isopren-Einheiten als Q-1, Q-2, Q-3 usw. bezeichnet. *Escherichia coli* enthält Ubichinone mit 1 bis 8 Isopren-Einheiten (Q-1 bis Q-8), Q-9 wurde in Fischen gefunden, Q-11 und Q-12 in Ratten. Bei den meisten Säugern jedoch, wie etwa dem Menschen, ist das Ubichinon (Coenzym) Q-10 die am häufigsten vorkommende Form. Ubichinone sind farblos bis schwach gelb in der Färbung und sind aufgrund ihrer lipophilen/hydrophoben Eigenschaften in organischen Lösungsmitteln und Fetten löslich.

Biologisch spielt Ubichinon eine wichtige Rolle in der Atmungskette des Energiestoffwechsels in den Mitochondrien der Zelle, In dieser Reaktionsabfolge wird das Ubichinon durch die Aufnahme eines einzigen Elektrons zu einem Semichinon mit einem freien Radikal, und durch die Aufnahme eines weiteren Elektrons zu Ubichinol reduziert.
Das Ubichinon kann durch die folgende Formel dargestellt werden:

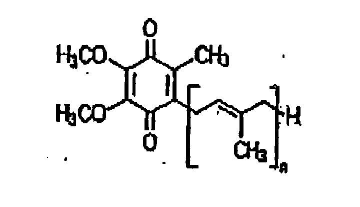

Neben seiner Rolle im Energiestoffwechsel wurden Eigenschaften des Ubichinons entdeckt, die seine Verwendung im medizinisch-pharmazeutischen und kosmetischen Bereich als auch als Nahrungsergänzungsmittel nahe legen. Dem Ubinchinon wird dabei eine Wirkung als vitanminähnlicher Stoff zugeschrieben, der verhindern kann, dass Körpergewebe von aggressiven Stoffen angegriffen werden, indem es wie die Vitamine C und E sogenannte freie Radikale "fängt" bzw. neutralisiert. Freie Radikale sind Substanzen, die unter besonderen Bedingungen, etwa bei UV-Strahlung oder beim Rauchen entstehen. Weil sie das Gewebe schädigen, werden sie für Krebs, Gefäßerkrankungen, weitere Leiden und auch mit negativen Erscheinungen der Alterung und verantwortlich gemacht. Darüber hinaus wird die Verwendung von Ubichinon zur Behandlung der Parkinson-Erkrankung diskutiert.

Durch seine lipophilen bzw. hydrophoben Eigenschaften ist es jedoch schwierig, Substanzen wie Biochinone und insbesondere Ubichinon in hydrophilen, z.B. wässrigen Zusammensetzungen, wie etwa in Getränken o.ä., zu verwenden, da es sich in derartigen Zusammensetzungen in nur sehr geringen Mengen löst.

Die Patentschrift DE-C-196 47 352 beschreibt ein nichtalkoholisches Getränk, z.B. Trinkwasser, mit 10mg/l bis 500mg/l Ubichinon Q-10, das durch die Verwendung des Lösungsvermittlers Polyoxyethylen-Sorbitanmonooleat (Polysorbat, E433) gelöst wurde.

In der Patentanmeldung DE-A-102 55 195 werden wasserlösliche micellare Konzentrate von fettartigen Substanzen beschrieben, die eine Kombination aus Phospholipiden oder Lecithinen und hochkonzentrierten wässrigen Lösungen aus Polyolen oder Kohlenhydraten enthalten.

Der vorliegenden Erfindung liegt die Aufgabe zugrunde, eine Verbindung einer wasserlöslichen Form von Biochinonen, insbesondere Ubichinonen, sowie ein Herstellungsverfahren dafür zur Verfügung zu stellen. Darüber hinaus stellt die vorliegende Erfindung die Verwendung dieser Verbindung sowie Zusammensetzungen, insbesondere pharmazeutische und kosmetische Formulierungen bzw. Nahrungsergänzungsmittel zur Verfügung. Diese Aufgaben werden durch die Merkmale der unabhängigen Ansprüchen 1, 13, 20, und 25 gelöst. Bevorzugte Ausführungsformen sind in den abhängigen Ansprüchen definiert.

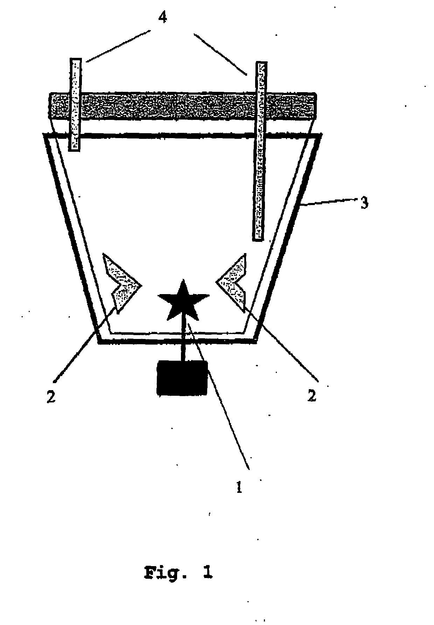

Die Fig.1 ist eine schematische Darstellung einer Vorrichtung für die Durchführung des erfindungsgemäßen Verfahrens. Hierin bezeichnet Bezugszeichen (1) ein Mahlwerk, Bezugszeichen (2) Prallvorrichtungen wie ein Gitter oder Platten, Bezugszeichen (3) eine Kühlvorrichtung, Bezugszeichen (4) eine Ent- bzw. Belüftungsvorrichtung zur Herstellung eines Vakuums bzw. einer Schutzgasatmosphäre und Bezugszeichen (5) eine Misch-und Mahlvorrichtung, in der die erfindungsgemäße Verbindung und Zusammensetzung hergestellt wird.

In einem bevorzugten Ausführungsbeispiel der Erfindung wird eine wasserlösliche, hydrophile Verbindung aus einem an einen Zucker gebunden Biochinon zur Verfügung gestellt.

In der vorliegenden Erfindung werden unter dem Begriff "Zucker" Kohlenhydrate verstanden, die zu der Gruppe der Einfachzucker, Mehrfachzucker oder der Zuckeralkohole gehören, wobei Zuckeralkohole besonders bevorzugt sind. Beispiele für erfindungsgemäße Zucker sind Dextrose, Fructose, Saccharose, Lactose, Maltose, Xylit, Mannitol, Sorbitol.

Das erfindungsgemäße Biochinon ist bevorzugt ein Ubichinon mit 1 bis 12 Isaprenoideinheiten (Q-1 bis Q-12) und ist besonders bevorzugt das Ubichinion Q-10.
Die Bindung des Biochinons an den Zucker beruht bevorzugt auf elektrostatischen Wechselwirkungen. Jedoch kann auch jeder andere, chemisch mögliche Bindungstyp in dem der erfindungsgemäßen wasserlöslichen Form eines Biochinons vorliegen.

Die maximale Löslichkeit des erfindungsgemäßen an einen Zucker gebundenen Biochinons in Pulverform in destilliertem Wasser bei 20°C ist bevorzugt bis zu 30 mg/l. In der fettigen Präparation für den Verbraucher kann die Löslichkeit, insbesondere bei höheren Zucker- oder Zuckeraustauschstoffgehalten ein Vielfaches davon betragen.

Die erfindungsgemäße Verbindung kann bevorzugt als feinkristallines Pulver, als Granulat, Emulsion oder in Gel- bzw. Geleeform vorliegen.

Die erfindungsgemäße Verbindung liegt in wässrigen Lösungen bevorzugt in kolloider Form vor, wobei die Kolloidpartikel bevorzugt eine Größe von 0,6 bis 0,7 nm haben.

Die Herstellung einer hydrophilen Form von Biochinon durch Bindung des Biochinonmoleküles an Zucker erfolgt bevorzugt ausschließlich mittels physikalischer Vorgänge, d. h. das resultierende Produkt kann frei von sonstigen chemischen Zusätzen sein. Die Bindung des Biochinon an den Zucker erfolgt bevorzugt mittels eines Verfahrens, in dem das Biochinon und Zucker miteinander in Kontakt gebracht werden und eine Reibung zwischen den beiden Substanzen erfolgt. Die Erzeugung von Reibung zwischen dem Biochinon und dem Zucker ist nicht auf ein bestimmtes Verfahren beschränkt.

Ein besonders bevorzugtes Ausführungsbeispiel des erfindungsgemäßen Verfahrens ist das Vermahlen eines wie oben definierten Biochinons mit einem wie vorher definierten Zucker mittels eines Mahlwerkes beliebiger Art, vorzugsweise unter hohem Druck und mit starker Geschwindigkeit zu einem feinkristallinen Pulver. Der Druck und die Mahlgeschwindigkeit können vom Fachmann nach bekannten Maßgaben eingestellt werden, die vom Mahlwerk abhängig sind.

Während des Herstellungsvorgangs wird in dieser Mahlvorrichtung, die nötige Reibungsenergie erzeugt, welche notwendig ist, damit sich die molekularen Teilchen elektrostatisch aufladen und eine elektrostatische Wechselwirkung eingehen.

Die Bindung zwischen dem Biochinon und dem Zucker kann gesteigert werden, wenn ein geeigneter Katalysator verwendet wird. Durch den Einfluss des Katalysators wird erreicht, dass die Bindung nicht nur stark genug wird, um eine Lösung in wässerigen Medien zu ermöglichen, sondern auch, dass diese Bindung über eine ausreichend lange Zeit erhalten bleibt. Als Katalysatoren eignen sich hierfür Bunt- oder Edelmetalle oder auch Legierungen davon, wobei einer großflächigen Anordnung während des Herstellungsvorganges bevorzugt ist, da so der katalytische Effekt erhöht werden kann.

Als Katalysatoren können bevorzugt Edelmetalle und Buntmetalle sowie ihre Legierungen eingesetzt werden. Bevorzugte Beispiele dafür sind Gold, Platin, Silber, Kupfer sowie Legierungen von diesen.

Um die im erfindungsgemäßen Verfahren entstehende Wärme zu entfernen, kann diese durch eine geeignete Kühlung der Vorrichtung abgeleitet werden.

Daneben ist es bevorzugt, den in der atmosphärischen Luft enthaltenen Sauerstoff während der Reaktion des Biochinons mit dem Zucker aus der Vorrichtung zu entfernen. Zu diesem Zweck kann das Verfahren unter Vakuum oder einer Schutzgasatmosphäre vollzogen werden. Dabei kann zunächst die atmosphärische Luft durch ein Schutzgas ersetzt und dieses sodann mittels einer Vakuumpumpe wiederum abgepumpt werden. Es entsteht dadurch eine nahezu sauerstofffreie Atmosphäre im Reaktionsraum.

Das erfindungsgemäße Verfahren wird bevorzugt in einer Vorrichtung mit einer Platte oder einem Gitter bestehend aus Bunt- oder Edelmetall oder einer Legierung aus solchen Metallen durchgeführt, welche während des Reibungsvorganges in die Pulvermasse eintaucht und so angeordnet ist, dass möglichst jedes Partikel damit in Berührung kommen kann.

Diese Vorrichtung ist bevorzugt ein Gefäß mit einem Doppelmantel und/oder einem isolierten Außenteil mit Kühlschlangen durch welche eine Kühlflüssigkeit geleitet werden kann, wobei diese Vorrichtung luftdicht abgeschlossen werden und Einlassöffnungen aufweisen kann, durch welche Luft abgepumpt und/oder Schutzgas zugeführt werden kann.

Das erfindungsgemäße Verfahren wird bevorzugt in einem Temperaturbereich von 10 bis 50°C, bevorzugter von 20 bis 30°C durchgeführt.

Das erfindungsgemäße Verfahren wird bevorzugt für Reaktionszeiten von 1 min bis 1 h, bevorzugter 5 bis 30 min, weiter bevorzugt von 10 bis 20 min durchgeführt.

Das in dem.erfindungsgemäßen Herstellungsverfahren erzeugte Produkt kann für pharmazeutische oder kosmetische Formulierungen oder als ein Nahrungsergänzungsmittel verwendet werden.

Für eine derartige Verwendung kann das z.B. nach dem Mahlvorgang gewonnene, feinkristalline Pulver in Wasser gelöst werden, wobei es bevorzugt ist, das Pulver zunächst in einer kleinen Menge härtefreiem, z.B. entionisiertem Wasser zu lösen. Dieses Konzentrat kann zur weiteren Verdünnung mit Wasser (z.B. Leitungswasser) oder wässrigen Zusammensetzungen oder Stoffgemischen weiterverarbeitet werden.

Ein weitere bevorzugte Verwendung besteht darin, dass die Pulvermasse zu einem Gelee oder Gel verarbeitet wird. In diesem Gelee bzw. Gel wird die Bindung zwischen dem Biochinon und dem Zucker über einen längeren Zeitraum aufrecht erhalten und ist vor allem als ein Ausgangsmaterial für die Herstellung von pharmazeutischen Formulierungen (Arzneimittel), Kosmetikprodukten und Nahrungsmitteln (wie z. B. Milchprodukten) einsetzbar. Dabei kann die Gel- oder Gallertmasse nach ihrem Erhalt getrocknet werden. Zur Trocknung sollten bevorzugt Temperaturen von 70°C nicht überschritten werden. Bei verringertem Druck lassen sich auch wesentlich geringere Trocknungstemperaturen (z.B. 30-50°C) einstellen.

Das Biochinon liegt in nach diesem Verfahren hergestellten Produkten in kolloider Form vor, wobei die Kolloidpartikel bevorzugt eine Größe von 0,6 bis 0,7 nm haben.

Die erfindungsgemäße Zusammensetzung mit einer wie vorher definierten Verbindung kann zusätzlich eine oder mehrere Substanzen ausgewählt aus der Gruppe der Stabilisatoren, Konservierungsmittel, Füllstoffe, Verdickungsmittel und Klebemittel enthalten.

Als Konservierungsmittel sind z.B. Emulgatoren, Sorbitmonostearat, Polyoxyethylenstearat, Ölsäure und deren übliche Derivate, Glycerylmonostearat, Pektine, Wachse, Harze, Paraffine u.dgl. einsetzbar, sofern sie die Funktionsfähigkeit der erfindungsgemäßen Präparation nicht beeinträchtigen. Als Klebemittel sind z.B. natürliche oder synthetische Latices und als Stabilisator Gummi arabicum einsetzbar.

Die Zusammensetzung kann z.B. als Pulver, Granulat, Molkeprodukt, Instant-Zubereitung (z.B. Brausetablette), Sirup, Saft, in Süß- und Backwaren, Kaugummi, Speiseeis, Joghurt, Pudding, Milch und allen flüssigen oder halbflüssigen Milchprodukten verwendet werden. Sie kann wasserlöslich sein und mit Aromastoffen versetzt sein.

Bevorzugt wird das erfindungsgemäße Produkt in eine Mischung aus Carboxymethylcellulose (CMC) und/oder Methylcellulose (MC) sowie Wasser, eingerührt. Hierbei entsteht eine gallertartige Masse, welche gut mit Wasser mischbar ist und über einen Zeitraum von mindestens einem halben Jahr haltbar und verarbeitbar ist.

Die weitere Verwendung der wie vorher hergestellten Gallertmasse für die Produktion von Instant-Zubereitungen kann beispielsweise mittels Sprüh- oder Gefriertrocknung und den dabei üblichen Füllstoffen wie Maltodextrin usw. erfolgen, wobei ein feinkristallines, besonders leicht wasserlösliches Pulver oder ein Granulat entstehen kann.

Aus dieser Pulverpräparation kann durch Zugabe von Wasser und einem Verdickungsmittel eine Gallertmasse hergestellt werden, wobei es sich bei den Verdickungsmittel beispielsweise um Cellulosederivate wie Carbooxymethylcellulose und/oder Methylcellulose (CMC/MC) handelt.

Die vorliegende Erfindung wird im Folgenden anhand von Beispielen anhand der Figur 1 weiter verdeutlicht, die jedoch den Umfang der Erfindung ihn keiner Weise beschränken. Die angegebenen Teile sind Gewichtsteile.

### Beispiel 1

Herstellung einer wasserlöslichen Präparation mit einem Gehalt von 5% Ubichinon in Form einer Gallertmasse:

**Folgende Bestandteile**

| | | | |
|---|---|---|---|
| 1. | saccharose | Teile | 450 |
| 2. | Ubichinon pur | Teile | 50 |

werden in einen Behälter eingefüllt. Zunächst wird die atmosphärische Luft durch Eintrag von Stickstoff oder Kohlendioxid ausgetragen, dann wird mittels einer Vakuumpumpe aus dem verschlossenen Gefäß Gas abgepumpt, sodass eine nahezu sauerstofffreie Situation in dem Reaktionsgefäß vorherrscht.

Nunmehr wird das Mahlwerk (Fig. 1) in Betrieb gesetzt, wobei eine Umdrehung von 2800 pro Minute erreicht wird. Das Mahlwerk wird 20 Minuten lang betrieben. Die während des Mahlvorganges entstehende Temperatur liegt zwischen 20 und 25°C. Zur Thermostatisierung wurde die angeschlossene Kühlvorrichtung (Fig. 2) entsprechend eingestellt.

Die vorliegende Pulvermischung besteht aus 10% Ubichinon und Saccharose, die miteinander verknüpft sind. Das Pulver lässt sich, ohne, dass sich Partikel voneinander trennen, gut in Wasser auflösen.

### Beispiel 2

### Fixierung der elektrostatischen Bindung:

In 460 Teilen entionisiertem Wasser werden 10 Teile Carboxymethylcellulose (CMC) vermischt mit 30 Teilen Saccharose unter kräftigen Rühren aufgelöst. Nach einer kurzen Quellzeit, sobald die Masse glatt geworden ist, wird das im Beispiel 1 hergestellte Pulver unter kräftigem Rühren eingebracht. Es entsteht eine gallertartige Masse mit einem Gehalt von 5% wasserlöslichem Ubichinon, welche sich nicht mehr ausmischt und unter bestimmten Lagerbedingungen, bevorzugt kühl (z.B. 4-8°C) und dunkel und im nicht gefrorenen Zustand mindestens 6 Monate haltbar und verarbeitbar bleibt.

Für die weitere Verarbeitung genügt es, die Gallertmasse mit normalem Trinkwasser anzuteigen und mit Wasser so weit zu verdünnen, dass es jeder anderen gewünschten, vorstehend genannten, Zubereitung beigefügt werden kann. Bei der Weiterverarbeitung, z.B. zu zuckerhaltigen Verzehrprodukten, bleibt die hydrophile Eigenschaft vollständig erhalten.

### Beispiel 3

### Herstellung einer pulverigen Form der Präparation.

Die nach Beispiel 2 hergestellte Präparation kann durch Wasserentzug zu einer trockenen Masse weiterverarbeitet werden. Hierbei ist darauf zu achten, dass die Temperatur des Mediums (Walze, Band, Trockenschrank oder Sprühturm) nicht über 70°C ansteigt.

Die Trocknung wird sehr langsam im Trockenschrank bei 70°C 5 Stunden lang durchgeführt (wobei der Auftrag nicht stärker als 0,5 cm sein soll) um eine gleichmäßige, funktionelle und mahlbare Trockenmasse zu erhalten.

Die Trockenmasse kann mittels Mahlwerk zu sehr feinem Pulver vermahlen werden, wobei dabei darauf zu achten ist, dass der Mahlvorgang die Masse nicht zu sehr erhitzt.

Das so gewonnene Pulver enthält 10% Ubichinon in hydrophiler Form und lässt sich ohne besondere Vorkehrungen in Wasser auflösen, wobei die Ubichinonanteile vollständig mit in Lösung gehen und diese Lösung auch stabil bleibt.

Für die Weiterverarbeitung z.B. zu Instantpräparaten, wie Brausetabletten ist es zweckmäßig, den Mahlgrad so klein wie möglich zu wählen. Je kleiner der Mahlgrad eingestellt wird, desto besser ist der Instant-Effekt.

Im Übrigen kann das Pulver auch in jedes andere wässrige Produkt, wie es vorstehend aufgeführt ist, durch einfaches Auflösen in Wasseranteil, eingebracht werden.

Die elektrostatische Stabilität dieser Form ist nahezu unbegrenzt.

### Beispiel 4

### Zubereitung in Form einer Emulsion.

Für eine Präparation mit einem Gehalt von 1,25% Ubichinon, z.B. zum direkten Genuss durch einen Verbraucher, kann folgendermaßen vorgegangen werden:

12,5 Xylit werden mit 1,25 kg Ubichinon im Reaktor wie in Beispiel 1 behandelt. Das so gewonnene Vorpräparat wird nun mit einer glatten und luftfreien Mischung aus 600 g Carboxymethylcellulose (CMC) und 100 g Methylcellulose (MC) in 90,0 l Wasser eingerührt.

Es entsteht eine genussfähige, cremig-viskose Flüssigkeit (Sirup) von neutralem Geruch und Geschmack. Diesen Sirup kann man mit Aromastoffen versetzen, z.B. mit Eierliköraroma, wobei für den Konsumenten der Eindruck entsteht, einen solchen Likör zu genießen.

Diese Präparation enthält in 2,5 ml (1 Teelöffel) 30 mg Ubichinon.

## Patentansprüche

1. Wasserlösliche hydrophile Verbindung aus einem an einen Zucker gebundenen Biochinon, inbesondere einem Ubichinon.

2. Verbindung nach Anspruch 1, wobei das Ubichinon ein Ubichinon Q-1 bis Q-12, insbesondere Q-10 ist.

3. Verbindung nach einem der vorhergehenden Ansprüche, wobei der Zucker ein Einfach- oder Mehrfachzucker aus der Gruppe Dextrose, Fructose, Saccharose, Lactose und Maltose oder ein Zuckeralkohol aus der Gruppe Xylit, Mannitol und Sorbitol ist.

4. Verbindung nach einem der Ansprüche 1 bis 3, wobei die Bindung auf zwischen dem Zucker und dem Biochinon auf elektrostatischer Wechselwirkung beruht.

5. Verbindung nach einem der Ansprüche 1 bis 4, der ein Katalysator aus der Gruppe Kupfer, Platin, Silber, Gold oder einer Legierung hiervon beigemischt ist.

6. Verbindung nach einem der vorhergehenden Ansprüche 1 bis 5, die in Form eines feinkristallinen Pulvers, eines Granulats oder in Gelform vorliegt.

7. Verbindung nach einem der vorhergehenden Ansprüche 1 bis 5, die in wässrigen Zusammensetzungen in kolloider Form vorliegt.

8. Verbindung nach einem der Ansprüche 1 bis 5, die zusammen mit Carboxymethylcellulose (CMC) und/oder Methylcellulose (MC) und/oder Maltodextrin zu einer gallertartigen Masse angesetzt ist.

9. Verfahren zur Herstellung einer Verbindung nach einem der Ansprüche 1 bis 4, bei welchem Reibungsenergie zwischen dem Biochinon und dem Zucker, insbesondere durch gemeinsames Vermahlen dieser Komponenten, zur Erzeugung der Bindung zwischen diesen erzeugt wird.

10. Verfahren nach Anspruch 9, das in einer sauerstofffreien Umgebung durchgeführt wird.

11. Verwendung einer Verbindung nach einem der Ansprüche 1 bis 8 in pharmazeutischen oder kosmetischen Formulierungen oder in Nahrungsergänzungsmitteln.
